(19) **Europäisches Patentamt / European Patent Office / Office européen des brevets**

(11) **EP 1 294 774 B1**

(12) **FASCICULE DE BREVET EUROPEEN**

(45) Date de publication et mention de la délivrance du brevet:
**13.08.2008 Bulletin 2008/33**

(51) Int Cl.:
**C08B 37/14** *(2006.01)* **C08L 5/14** *(2006.01)*
**A23L 1/0526** *(2006.01)*

(21) Numéro de dépôt: **01943600.5**

(22) Date de dépôt: **12.06.2001**

(86) Numéro de dépôt international:
**PCT/FR2001/001809**

(87) Numéro de publication internationale:
**WO 2001/098369 (27.12.2001 Gazette 2001/52)**

(54) **PROCEDE DE PREPARATION DE CAROUBE SOLUBLE ET SES UTILISATIONS**

VERFAHREN ZUR HERSTELLUNG VON LÖSLICHEM JOHANNISBROTGUMMI UND DESSEN VERWENDUNGEN

PROCESS OF MANUFACTURE OF SOLUBLE CAROB AND ITS USES

(84) Etats contractants désignés:
**AT BE CH CY DE DK ES FI FR GB GR IE IT LI LU MC NL PT SE TR**

(30) Priorité: **19.06.2000 FR 0007802**

(43) Date de publication de la demande:
**26.03.2003 Bulletin 2003/13**

(73) Titulaire: **DANISCO A/S**
**1411 Copenhagen K. (DK)**

(72) Inventeurs:
• **DELPRATO, François**
**F-95310 Saint-Ouen l'Aumone (FR)**
• **TIEFENTHALER, Karl, Heinrich, Oskar**
**CH-8280 Kreuzlingen (CH)**
• **GORON, Eric**
**CH-8280 Kreuzlingen (CH)**

• **VASLIN, Sophie**
**F-92210 Saint-Cloud (FR)**

(74) Mandataire: **Touati, Catherine et al**
**Cabinet Plasseraud**
**52 rue de la Victoire**
**75440 Paris Cedex 09 (FR)**

(56) Documents cités:
**FR-A- 974 862        US-A- 3 855 149**

• **S.E.GAISFORD ET AL.: "A comparison betwween the hot and cold water soluble fractions of two Locust Bean Gum samples" CARBOHYDRATE POLYMERS, vol. 6, 1986, pages 423-442, XP002162511**
• **PATENT ABSTRACTS OF JAPAN vol. 2000, no. 02, 29 février 2000 (2000-02-29) & JP 11 308973 A (NISSHIN OIL MILLS LTD:THE), 9 novembre 1999 (1999-11-09)**

**Description**

La présente invention concerne le procédé de préparation d'une gomme de caroube présentant une masse moléculaire moyenne en poids ($\overline{M}_w$) comprise entre $2,5.10^5$ et $1,5.10^6$ g/mol et dont au moins 60 % en poids de ladite gomme est soluble en milieu aqueux à une température égale ou inférieure à 60°C et son utilisation.

Elle concerne également une composition gélifiante comprenant ladite gomme de caroube et au moins un hydrocolloïde.

Diverses industries telles que les industries cosmétique, tinctoriale, alimentaire, pétrolière, des produits d'entretien recherchent des agents de texture qui modifient les propriétés rhéologiques des phases liquides, notamment des phases aqueuses.

Les modifications de rhéologie des phases aqueuses sont habituellement obtenues par des dérivés de glucides d'origine végétale ou animale.

Le choix de ces dérivés de glucides dépend par exemple de leur prix, de leur disponibilité, des conditions dans lesquelles on peut les utiliser, et des types de composés dans lesquels ils peuvent convenir.

Ainsi, les galactomannanes qui sont des polysaccharides non ioniques extraits de l'albumen de graines de légumineuses dont ils constituent le glucide de réserve, sont souvent employés comme agent de texture.

Parmi les galactomannanes les plus employés, on peut citer la gomme de caroube. Elle est extraite des graines de caroubier *(Ceratonia siliqua L.)* qui est un arbre à feuillages persistants originaire de Syrie et d'Asie mineure, mais cultivé sur tout le littoral méditerranéen.

La gomme de caroube est composée d'une chaîne principale constituée d'unités de D-mannopyranose liées en β(1-4) portant des branchements latéraux constitués d'une seule unité de D-galactopyranose en position α(1-6).

La gomme de caroube est, en général, connue pour ses propriétés texturantes, et plus particulièrement pour ses propriétés épaississantes et stabilisantes.

Elle est plus particulièrement intéressante car, de par sa nature, elle est compatible avec d'autres composés notamment des hydrocolloïdes, et donc peut être utilisée en association avec ceux-ci pour produire des effets synergiques en terme de texture. Par exemple, en association avec la gomme xanthane ou les carraghénanes, des gels de différentes forces et de consistance plus ou moins élastique peuvent être obtenus.

Cependant, en milieu aqueux la gomme de caroube native ne présente une solubilité satisfaisante, c'est-à-dire supérieure à 60% en poids de la gomme, qu'à des températures supérieures à 80°C.

Ainsi, pour des raisons pratiques et économiques, aujourd'hui les industriels cherchent de plus en plus à remplacer la gomme de caroube native par des gommes de caroubes pouvant présenter une bonne solubilité à des températures inférieures aux températures de solubilisation habituelles des gommes de caroubes natives.

La présente invention a pour but de fournir une gomme de caroube ayant les propriétés analogues à celles des caroubes natives (par exemple en termes de texturation, de compatibilité avec d'autres composés) et une bonne solubilité à des températures bien inférieures aux températures de solubilisation habituelles des gommes de caroubes natives.

Elle a encore pour but de proposer une gomme de caroube, qui, à concentration constante, permet d'atteindre une gamme de viscosité variable.

Dans ce but la présente invention a pour objet un procédé de préparation de la gomme de caroube présentant une masse moléculaire moyenne en poids ($\overline{M}_w$) comprise entre $2,5.10^5$ et $1,5.10^6$ g/mol et dont au moins 60 % en poids de ladite gomme est soluble en milieu aqueux à une température égale ou inférieure à 60°C.

L'invention a encore pour objet des compositions gélifiantes comprenant la gomme de caroube de type ci-dessus et au moins un hydrocolloïde.

Enfin, l'invention concerne les formulations alimentaires comprenant des compositions gélifiantes mentionnées précédemment.

Dans le cadre de la présente invention, l'expression « solubilité en milieu aqueux à une température égale ou inférieure à 60°C » signifie qu'à une température égale ou inférieure à 60°C, en milieu aqueux, la gomme de caroube développe 60% de la viscosité qu'elle aurait développée si elle avait été mise en solution à des températures supérieures à 80°C.

Au sens de l'invention, «milieu aqueux» désigne un milieu qui est au moins partiellement constitué d'eau.

Ainsi, la gomme de caroube présente une masse moléculaire moyenne en poids ($\overline{M}_w$) comprise entre $2,5.10^5$ et $1,5.10^6$ g/mol et au moins 60 % en poids de ladite gomme est soluble en milieu aqueux à une température égale ou inférieure à 60°C.

Dans ces conditions de température, la solubilité de la gomme peut être avantageusement d'au moins 70% en poids, et de préférence d'au moins 80% en poids de ladite gomme.

Ces niveaux de solubilités - c'est-à-dire au moins 60% en poids, avantageusement au moins 70% en poids, et de préférence au moins 80% en poids de la gomme - peuvent être atteints plus particulièrement à température une inférieure à 60°C, avantageusement comprise entre 10°C et 45°C, de préférence comprise entre 15°C et 30°C.

Ces niveaux de solubilité peuvent aussi être atteints à une température pouvant varier de 20°C à 25°C.

La masse moléculaire moyenne en poids ($\overline{M}_w$) de la gomme est avantageusement comprise entre $2,5.10^5$ et $1.10^6$ g/mol, et de préférence entre $2,5.10^5$ et $6.10^5$ g/mol.

Elle peut être mesurée par chromatographie par perméation de gel (GPC). On utilise dans ce cas des solutions standard de polymères synthétiques pour la calibration.

Elle peut également être déterminée par la diffusion de la lumière.

La mesure de la viscosité peut aussi donner une indication sur la masse moléculaire moyenne en poids ($\overline{M}_w$). Ainsi, à 25°C, une solution aqueuse à 1% de la gomme de caroube selon l'invention présente une viscosité comprise entre 15 et 2000 mPa.s (mesurée à 20 tr/min). La viscosité, pour une telle solution, est de préférence comprise entre 15 et 1000 mPa.s.

La viscosité peut être mesurée à l'aide d'un viscosimètre Brookfield DV-III, à 25°C et 20 tr/min.

La masse moléculaire moyenne en poids ($\overline{M}_w$) de la gomme de caroube est telle que sa viscosité intrinsèque [η] soit supérieure à 2,3 dl/g.

La relation entre la viscosité intrinsèque [η] et la masse moléculaire moyenne en poids ($\overline{M}_w$) peut être établie de la manière suivante (A. Sabater de Sabates, 1979, Thèse de doctorat, Université d'Orsay, ENSIA, Massy - France) :

$$[\eta] = 1,24.10^{-4} \times \overline{M}_w^{\,0,8}$$

Cette viscosité est mesurée au moyen d'un viscosimètre en U de type Übbelhode.

Le rapport massique D-mannopyranose/D-galactopyranose (M/G) de la caroube est compris entre 1 et 5, de préférence entre 2 et 4. La connaissance de ce rapport constitue un des moyens de caractériser l'échantillon bien qu'il n'informe pas sur la distribution statistique des branchements D-galactopyranoses sur la chaîne principale.

On obtient de la gomme de caroube présentant les caractéristiques précitées par réduction de la masse moléculaire moyenne en poids ($\overline{M}_w$) de la gomme native. Cette réduction est en général obtenue par la rupture des liaisons glycosiques dans le but de produire des chaînes plus courtes qui soient sensiblement identiques, du point de vue chimique, à la gomme native.

La réduction de la masse moléculaire moyenne en poids ($\overline{M}_w$) confère certains avantages à la gomme. Elle permet, par exemple, d'obtenir une caroube dont les propriétés texturantes et en particulier les propriétés épaississantes et stabilisantes peuvent être ajustées. Elle permet, en outre, d'obtenir une gomme de caroube capable de former des pseudo-gels et/ou ayant des propriétés émulsifiantes, ou ce qui la distingue de la gomme native.

L'invention a pour objet le procédé de préparation de la gomme de caroube telle que décrite plus haut.

Ce procédé comporte les étapes suivantes :

*(i)* on hydrate l'endosperme de la gomme de caroube ;
*(ii)* on procède à un séchage et un broyage simultanés de l'endosperme hydraté ;
*(iii)* à l'issue de l'étape *(ii)* on réduit la masse moléculaire moyenne en poids de la caroube par la dépolymérisation de celle-ci.

Dans l'étape *(i)* l'hydratation de l'endosperme de la gomme de caroube est réalisée pendant un temps suffisant pour atteindre un taux d'hydratation compris entre 50 et 90 %.

Le taux d'hydratation peut se calculer, par exemple, simplement en mesurant l'extrait sec de l'endosperme.

A l'issue de l'étape *(ii)*, la caroube est sous forme de poudre avec une teneur en eau qui est avantageusement comprise entre 6 et 12%.

La granulométrie de cette poudre peut varier selon les conditions opératoires. Elle peut varier par exemple de 20 à 200 μm.

La mesure de la granulométrie peut s'effectuer par diffraction laser, par exemple, à l'aide d'un granulomètre laser Malvern Mastersizer® 2000.

L'opération de séchage - broyage est plus particulièrement effectuée à une température comprise entre 30°C et 90°C.

Cette opération peut être réalisée dans tout type d'appareil permettant un broyage et un séchage simultanés comme par exemple un moulin à marteaux ou aiguilles.

La dépolymérisation de la caroube peut s'effectuer par oxydation, par voie enzymatique, par hydrolyse acide, sous l'effet de pressions et de températures élevées et en présence d'un oxydant, ou par traitements physiques comme par exemple par exposition à des rayonnements de type gamma.

La dépolymérisation est avantageusement réalisée par oxydation. L'oxydation est de préférence effectuée en milieu alcalin, en présence d'un agent de type de la famille des peroxydes tels que l'acide péracétique et $H_2O_2$.

Le temps de la réaction de dépolymérisation est en fonction de la masse moléculaire moyenne en poids finale souhaitée.

La dépolymérisation peut s'effectuer dans un réacteur muni d'un système de mélange adapté à la manipulation de poudre fine, c'est-à-dire de granulométrie de l'ordre de 20 à 200 $\mu$m, et ce de manière à éviter la formation de grumeaux. A ce titre, on peut citer de manière non limitative les réacteurs de type Lödige, les mélangeurs à ruban.

Après la dépolymérisation, le cas échéant, on neutralise l'excès de l'alcalinité par adjonction, par exemple, d'hydroxyde d'ammonium, ou d'un acide tel que l'acide acétique, l'acide citrique, l'acide phosphorique ou acide sulfurique.

La caroube dépolymérisée peut alors être séchée. Ce séchage peut être effectué dans tout type d'appareil évitant la formation d'agglomérats. On peut utiliser, par exemple, une Turbosphère, un moulin à aiguilles, un flash.

La gomme de caroube finale se présente de préférence sous la forme d'une poudre. Sa teneur en eau est avantageusement comprise entre 6 et 12 %.

Les étapes peuvent être effectuées dans un ordre quelconque. Toutefois, elles sont de préférence effectuées dans l'ordre indiqué plus haut.

La gomme de caroube ainsi obtenue présenter les caractéristiques physico-chimiques parfaitement compatibles avec les définitions de la gomme de caroube telles que décrites dans le "Food Chemical Codex", 4ème édition, page 768 et dans la Directive de l'Union Européenne. EU n°98/86/CE du 11 novembre 1998.

La gomme de caroube ainsi obtenue peut être utilisée en tant qu'agent de texture, notamment en tant qu'épaississants, stabilisants, gélifiants (capable de former des pseudo-gels) et/ou émulsifiants dans le domaine de la cosmétique, des industries tinctoriale, alimentaire, celle du pétrole et celle des produits d'entretien.

Ladite gomme est destinée plus particulièrement au domaine de l'alimentaire.

En association avec d'autres composés, notamment des hydrocolloïdes, cette gomme de caroube peut former un gel dont les propriétés physiques (point de fusion, force du gel) peuvent être contrôlées.

A ce stade il convient de définir le terme "gel". Par "gel", dans le cadre de la présente invention, on désigne un pseudo-solide (comportement proche du solide), résultant de l'association, au moins partielle, de chaînes de polysaccharides dispersées dans un liquide. Dans un domaine de fréquences de sollicitation $\omega$, les gels pseudo-solides sont en général caractérisés en ce qui concerne leur composante solide par un module élastique $G'(\omega)$ appelé également module de conservation, et en ce qui concerne leur composante liquide ou visqueuse par un module visqueux $G''(\omega)$ appelé également module de perte.

Les grandeurs mécaniques $G'(\omega)$ et $G''(\omega)$ peuvent être mesurées à l'aide d'un rhéomètre à déformation imposée et fonctionnant en mode oscillatoire. A titre indicatif et non limitatif, on peut citer par exemple un rhéomètre Rheo-Fluid Spectrometer®.

$G'(\omega)$ et $G''(\omega)$ peuvent aussi être mesurés à l'aide d'un rhéomètre à contrainte imposée et fonctionnant en mode oscillatoire. A titre indicatif, on peut citer par exemple un rhéomètre AR1000® (TA Instruments).

Le principe de la mesure consiste à déterminer dans un premier temps le domaine de déformation mécanique réversible dans lequel la réponse du gel à la sollicitation mécanique est linéaire en fonction de ladite déformation. Dans un second temps, le gel est soumis à une valeur fixe de déformation mécanique comprise dans le domaine linéaire précédemment déterminé. C'est alors que le rhéomètre procède à un balayage en fréquence $\omega$.

La réponse en contrainte du gel qui est en phase avec la déformation donne accès au module élastique $G'(\omega)$. $G'(\omega)$ correspond à l'énergie emmagasinée par le gel sous forme élastique et est récupérable.

La réponse en contrainte du gel qui est en déphasage d'un angle 90° avec la déformation donne accès au module visqueux $G''(\omega)$. $G''(\omega)$ correspond à l'énergie dissipée par l'écoulement visqueux et est irrécupérable.

Un gel est dit vrai lorsque, dans tout le domaine de fréquences de sollicitation ($\omega$) balayées le rapport $G'/G''$ est supérieur ou égal à 10 et lorsque la valeur de $G'(\omega)$ est supérieure ou égale à 10 Pa.

De la même manière, un gel est dit pseudo-gel lorsque dans tout le domaine de fréquences de sollicitation ($\omega$) balayées le rapport $G'/G''$ est supérieur ou égal à 5 et lorsque la valeur de $G'(\omega)$ est supérieure ou égale à 1 Pa.

Un autre aspect de la présente invention est relatif aux compositions gélifiantes comprenant de la gomme de caroube ainsi obtenue avec au moins un hydrocolloïde.

De manière tout à fait inattendue, il a été constaté que cette gomme de caroube seule ou en association avec au moins un hydrocolloïde pouvait conduire à un pseudo-gel à une température égale ou inférieure à 60°C. Il a également été constaté que, comme pour la gomme de caroube native, l'association de la caroube ainsi obtenue avec au moins un hydrocolloïde pouvait donner des gels vrais par chauffage à une température égale ou supérieure à 80°C.

Parmi les hydrocolloïdes, on peut citer à titre non limitatif la gomme xanthane, les carraghénanes, l'agar, l'agar danois (Danish agar), la gomme gellane. De préférence, l'hydrocolloïde est la gomme xanthane.

Dans ces compositions le rapport massique entre la gomme de caroube telle que définie précédemment et le (s) hydrocolloïde(s) est (sont) choisi(s) entre 5/95 et 95/5, avantageusement entre 20/80 et 80/20, et de préférence entre 40/60 et 60/40.

Selon un mode de réalisation de l'invention, ce rapport massique est 50/50.

Ces compositions peuvent être obtenues par simple mélange des composants, à savoir la caroube et le ou les hydrocolloïdes. Les composants peuvent être mélangés sous la forme de poudre et mis en solution par la suite. Ils peuvent aussi être mélangés sous forme de solutions. Il est également envisageable d'ajouter l'un des composants sous forme de poudre à l'autre composant qui se trouve en solution. Dans ce dernier cas, il est important d'éviter la formation d'agglomérats lors du mélange.

L'invention concerne, en outre, un procédé de gélification d'une phase aqueuse, caractérisé en ce que l'on forme un pseudo-gel par ajout d'une composition gélifiante telle que définie précédemment à ladite phase, à une température égale ou inférieure à 60°C, plus particulièrement inférieure à 60°C, avantageusement comprise entre 10°C et 45°C, de préférence comprise entre 15°C et 30°C, et après un temps de repos suffisant.

Dans un mode de réalisation préféré de l'invention l'ajout de la composition gélifiante peut se faire à une température pouvant varier de 20°C à 25°C.

Il est à noter que dans le mode de réalisation préféré de l'invention, l'ajout de la composition gélifiante ainsi que la formation du pseudo-gel ont lieu à une température pouvant varier de 20°C à 25°C.

L'invention s'étend également au procédé de gélification d'une phase aqueuse, caractérisé en ce que l'on forme un gel vrai par ajout d'une composition gélifiante telle que définie précédemment à ladite phase, à une température égale ou supérieure à 80°C, et un temps de repos suffisant.

Etant donné que pour former un gel vrai, l'ajout de la composition gélifiante à la phase aqueuse se fera à des températures égales ou supérieures à 80°C, une étape de refroidissement sera éventuellement nécessaire.

L'homme du métier est en mesure de déterminer le temps de repos adapté selon les valeurs de G'($\omega$) et G"($\omega$) souhaitées.

Ainsi, on a pu constater que par exemple des solutions à 1 % en poids/poids de la composition gélifiante dans l'eau distillée à 25°C, et à une fréquence de 1 Hz, conduisent à des valeurs de G'($\omega$) comprises entre 1 et 1000 Pa, de préférence entre 10 et 1000 Pa, avec un rapport G'/ G" supérieur à 5.

La quantité de la composition gélifiante pouvant être mise en oeuvre dépendra de la phase aqueuse à gélifier. Celle-ci peut représenter de 0,01 à 10 %, avantageusement de 0,5 à 2 %, et de préférence de 0,8 à 1,5 % en poids de la phase gélifiée.

La composition gélifiante peut être introduite indifféremment soit sous forme solide soit sous forme d'une solution aqueuse.

Ces compositions gélifiantes peuvent être utilisées dans les industries pétrolière, agrochimique, alimentaire, papetière, textile, ainsi que dans les peintures et les nettoyants ménagers ou industriels.

Plus particulièrement, les compositions gélifiantes sont destinées à des formulations alimentaires.

Parmi les formulations alimentaires dans lesquelles l'utilisation de telles compositions sont adaptées, on peut citer par exemple des compositions de types gelées, flan, crème renversée, aspic, chauds froids de volailles, bavarois, yaourts, crèmes glacées, sorbets, crèmes brûlées et boissons.

Des exemples concrets mais non limitatifs de l'invention vont maintenant être donnés.

**EXEMPLES**

Dans les exemples qui vont suivre, la viscosité intrinsèque ainsi que la masse moléculaire moyenne en poids ($\overline{M}_w$) sont déterminées de la manière suivante :

*Mesure de la viscosité intrinsèque*

Une solution à 1% poids/poids de la caroube selon l'invention est préparée par agitation vigoureuse dans de l'eau déminéralisée. La solution est alors laissée au repos à environ 25°C pendant 24 h. Par centrifugation 1 h à 14000 tr/min, on récupère le surnageant que l'on filtre sur de la laine de verre. Cette solution mère est alors utilisée pour préparer des solutions de concentrations inférieures par dilution dans de l'eau déminéralisée.

Les viscosités réduites, puis spécifiques, sont alors calculées à 25°C avec un viscosimètre en U de type Übbelhode en mesurant les temps de chute.

Enfin, les viscosités intrinsèques [$\eta$] sont déterminées par extrapolation à concentration nulle :

- soit par la méthode de Huggins en traçant la courbe « viscosité spécifique / concentration » en fonction de la concentration ;
- soit par la méthode de Kraemer en traçant la courbe « (logarithme népérien de la viscosité réduite) / concentration » en fonction de la concentration.

Les deux méthodes conduisent au même résultat.

*Mesure de la masse moléculaire en poids*

La masse moléculaire en poids est déterminée par chromatographie de perméation sur gel en utilisant des solutions étalons d'oxyde de polyéthylène comme standards de calibration.

## Exemple 1 : Préparation de gomme de caroube obtenue à l'aide du procédé selon l'invention

En suivant les étapes *(i)* à *(iii)* du procédé décrit dans le texte (dépolymérisation par oxydation alkaline en présence de soude NaOH et de peroxyde d'hydrogène $H_2O_2$, neutralisation avec de l'acide acétique $CH_3CO_2H$, temps de réaction de 100 minutes à 70°C) dans l'ordre indiqué, on obtient une gomme de caroube présentant les caractéristiques suivantes :

- **-** teneur en eau : 11,2% ;
- **-** viscosité Brookfield à 25°C et 20 tr/min d'une solution à 2% poids/poids dans de l'eau déminéralisée :

  - **-** après 24 h et mise en solution à 25°C : 150 mPa.s ;
  - **-** après 24 h et mise en solution à 80°C : 190 mPa.s ;

  D'où une solubilité de 79% ;
- **-** viscosité intrinsèque : $[\eta] = 3,7$ dl/g ;
- **-** masse moléculaire moyenne en poids : $\overline{M}_w = 4,5.10^5$ g/mol ;
- **-** tension superficielle à l'équilibre d'une solution à 0,5% poids/poids dans de l'eau distillée : y = 39 mN/m (mesurée à 25°C par l'intermédiaire d'un tensiomètre à goutte tombante de type LAUDA TVT 1).

## Exemple 2 (comparatif) : Préparation de gomme de caroube uniquement dépolymérisée

En effectuant uniquement l'étape de dépolymérisation *(iii)* d'une gomme de caroube native, et sans passer préalablement à la dépolymérisation par les étapes *(i)* et *(ii)*, on obtient une gomme de caroube présentant une viscosité, après mise en solution à une température égale ou supérieure à 80°C, similaire à la gomme de caroube de l'exemple 1. Toutefois, le niveau de solubilité de cette gomme est sensiblement inférieur à 60% en poids. Cette gomme présente les caractéristiques suivantes :

- **-** viscosité Brookfield à 25°C et 20 tr/min d'une solution à 2% poids/poids dans de l'eau déminéralisée :

  - **-** après 24 h et mise en solution à 25°C : 57 mPa.s ;
  - **-** après 24 h et mise en solution à 80°C : 243 mPa.s ;

D'où une solubilité de 23%.

## Exemple 3 : Composition gélifiante selon l'invention

La composition gélifiante de l'exemple 3 est préparée en mélangeant, dans un rapport en poids 50/50, les deux poudres suivantes : la gomme de caroube de l'exemple 1 et de la gomme xanthane (Rhodigel™ 200, commercialisé par la société Rhodia).
Après mise en solution de cette composition gélifiante par agitation vigoureuse pendant 10 minutes dans un milieu aqueux à 25°C, et après un temps de repos de 24 h à une température de l'ordre de 25°C on obtient des pseudo-gels dont les caractéristiques sont les suivantes :

- **-** pour une concentration totale en composition gélifiante de 1% poids/poids dans de l'eau déminéralisée, les modules $G'(\omega)$ et $G''(\omega)$ sont respectivement de 40 et 8 Pa ;
- **-** pour une concentration totale en composition gélifiante de 1 % poids/poids dans du lait écrémé (solution à 10% poids/poids de lait écrémé en poudre dans de l'eau déminéralisée), les modules $G'(\omega)$ et $G''(\omega)$ sont respectivement de 100 et 20 Pa.

Pour la même composition gélifiante, après mise en solution par agitation vigoureuse pendant 10 minutes dans un milieu aqueux à une température supérieure ou égale à 80°C, et après un temps de repos de 24 h à une température de l'ordre de 25°C, on obtient des gels vrais de caractéristiques suivantes (concentration totale en composition gélifiante de 1 % poids/poids dans une solution à 1 % poids/poids de KCl) :

- les modules G'($\omega$) et G"($\omega$) sont respectivement de 140 et 7 Pa ;
- les températures de gélification et de fusion sont respectivement de 54°C et 56°C (ces températures sont déterminées au croisement des courbes G' et G" en fonction de la température ; variation à -2°C/min pour la gélification et à +2°C/min pour la fusion, dans le domaine de déformation mécanique réversible du gel, à une fréquence de 1 Hz) ;
- la force du gel pour une distance d'enfoncement de 10 mm est de 80 g/cm$^2$ (déterminée à l'aide d'un texturomètre de type TA-XT2® pour une vitesse d'enfoncement du piston de 0.5 mm/s ; cylindre de section 1 cm$^2$).

A titre de comparaison, en suivant le même mode opératoire que celui décrit précédemment (mise en solution du mélange des poudres par agitation vigoureuse pendant 10 minutes dans un milieu aqueux à une température égale ou supérieure à 80°C, temps de repos de 24 h à une température de l'ordre de 25°C), on obtient avec un mélange caroube native (Meypro-Fleur™ 200, commercialisé par la société Meyhall AG) / gomme xanthane (Rhodigel™ 200, commercialisé par la société Rhodia) (rapport en poids 50/50), des gels vrais de caractéristiques suivantes (concentration totale en gommes de 1 % poids/poids dans une solution à 1 % poids/poids de KCl) :

- les modules G'($\omega$) et G"($\omega$) sont respectivement de 200 et 10 Pa ;
- les températures de gélification et de fusion sont respectivement de 62°C et 67°C ;
- la force du gel pour une distance d'enfoncement de 10 mm est de 83 g/cm$^2$.

On peut donc constater que l'interaction entre la gomme de caroube de l'invention obtenue à l'aide du procédé et la gomme de xanthane est fortement favorisée par rapport à la gomme de caroube native (en effet pour la même quantité de gomme de caroube selon l'exemple 1 et de gomme de caroube native, on obtient la même force de gel, bien que la viscosité Brookfield développée par la gomme de caroube native soit nettement supérieure : 3000 mPa.s à une concentration de 1 % poids/poids ; mise en solution à une température supérieure à 80°C ; viscosité Brookfield mesurée à 25°C et 20 tr/min).

### Exemple 4 : Utilisation d'une composition gélifiante dans les formulations alimentaires de types flan

Flan à la vanille

| Ingrédients | |
|---|---|
| Sucre | 12,0% |
| Composition gélifiante[1] | 0,80% |
| Arôme vanille | 0,05% |
| Colorant | 0,05% |
| NaCl | 0,02% |
| Lait demi-écrémé | complément jusqu'à 100% |

Agent gélifiant[1] = il s'agit de la composition gélifiante de l'exemple 3 : mélange gomme de caroube de l'exemple 1 avec gomme xanthane (Rhodigel™ 200, commercialisé par la société Rhodia) dans un rapport en poids 50/50.

*Recette*

Mélanger la composition gélifiante aux autres ingrédients secs ; Chauffer le lait à 85°C ;
Introduire le mélange des poudres et agiter à 85°C, 15 minutes à 1000 tr/min ;
Laisser refroidir et reposer une nuit au réfrigérateur à 4°C-6°C.

*Caractérisation du flan*

On obtient un gel élastique, de force de gel modérée (30 g/cm$^2$ à 10 mm) et de qualités organoleptiques convenables.
Si dans la composition gélifiante on remplace la gomme de caroube de l'exemple 1 par de la gomme de caroube native, on obtient un flan plus ferme et moins agréable en bouche..

**Revendications**

1. Procédé de préparation d'une gomme de caroube comportant les étapes suivantes :

   *(i)* on hydrate l'endosperme de la gomme de caroube ;
   *(ii)* on procède à un séchage et un broyage simultanés de l'endosperme hydraté ;
   *(iii)* à l'issue de l'étape *(ii)* on réduit la masse moléculaire moyenne en poids de la caroube par une réaction de dépolymérisation de celle-ci.

2. Procédé selon la revendication 1, **caractérisé en ce que** dans l'étape *(i)* l'hydratation de l'endosperme de la gomme de caroube est réalisée pendant un temps suffisant pour atteindre un taux d'hydratation comprise entre 50 et 90 %.

3. Procédé selon l'une des revendications 1 ou 2, **caractérisé en ce qu'**à l'issue de l'étape *(ii)*, la caroube est sous forme de poudre avec une teneur en eau qui est avantageusement comprise entre 6 et 12%.

4. Procédé selon l'une quelconque des revendications 1 à 3, **caractérisé en ce que** l'opération de séchage - broyage est effectuée à une température comprise entre 30°C et 90°C.

5. Procédé selon l'une quelconque des revendications 1 à 4, **caractérisé en ce que** la dépolymérisaton de l'étape *(iii)* est réalisée par oxydation.

6. Utilisation d'une gomme de caroube obtenue selon l'une quelconque des revendications 1 à 5 en tant qu'épaississants, stabilisants, gélifiants (capable de former des pseudo-gels) et/ou émulsifiants dans le domaine de la cosmétique, des industries tinctoriale, alimentaire, celle du pétrole et celle des produits d'entretien.

7. Composition gélifiante comprenant de la gomme de caroube obtenue selon l'une quelconque des revendications 1 à 5 avec au moins un hydrocolloïde.

8. Composition gélifiante selon la revendication précédente, **caractérisée en ce que** l'hydrocolloïde est choisi parmi la gomme xanthane, les carraghénanes, l'agar, l'agar danois (Danish Agar), la gomme gellane.

9. Composition gélifiante selon l'une des revendications 7 ou 8, **caractérisée en ce que** le rapport massique entre la gomme de caroube et le(s) hydrocollorde(s) est comprise entre 5/95 et 95/5, avantageusement entre 20/80 et 80/20 , et de préférence entre 40/60 et 60/40.

10. Composition gélifiante selon la revendication 9, **caractérisée en ce que** le rapport massique entre la gomme de caroube et le(s) hydrocolloïde(s) est 50/50.

11. Procédé de gélification d'une phase aqueuse de gélification, **caractérise en ce que** l'on forme un pseudo-gel par ajout d'une composition gélifiante selon l'une quelconque des revendications 1 à 10 à ladite phase, à une température égale ou inférieure à 60°C, plus particulièrement inférieure à 60°C, avantageusement comprise entre 10°C et 45°C, de préférence comprise entre 15°C et 30°C, et après un temps de repos suffisant.

12. Procédé de gélification d'une phase aqueuse selon la revendication précédente, **caractérisé en ce que** l'on forme un pseudo-get par ajout d'une composition gélifiante selon l'une quelconque des revendications à à ladite phase, à une température variant de 20°C à 25°C.

13. Procédé de gélification d'une phase aqueuse, **caractérisé en ce que** l'on forme un gel vrai par ajout d'une composition gélifiante selon l'une quelconque des revendications 7 à 10 à ladite phase, à une température égale ou supérieure à 80°C, et un temps de repos suffisant.

14. Utilisation d'une composition gélifiante selon l'une quelconque des revendications 7 à 10, dans les industries pétrolière, agrochimique, alimentaire, papetière, textile, ainsi que dans les peintures et les nettoyants ménagers ou industriels.

15. Utilisation d'une composition gélifiante selon la revendication 14 dans les formulations alimentaires de types gelées, flan, crème renversée, aspic, chauds froids de volailles, bavarois, yaourts, crèmes glacées, sorbets, crèmes brûlées, boissons.

**Claims**

1.  A method of preparing a carob bean gum, comprising the following steps :

    (i) the endosperm of the carob bean gum is hydrated;
    (ii) the hydrated endosperm is simultaneously dried and ground; and
    (iii) after step (ii), the weight-average molecular mass of the carob is reduced by depolymerizing the latter.

2.  The method as claimed in claim 1, **characterized in that** in step (i), the hydration of the carob bean gum endosperm is carried out for a time long enough to achieve a degree of hydration of between 50 and 90%.

3.  The method as claimed in either of claims 1 or 2, **characterized in that** after step (ii), the carob is in the form of a powder with a water content which is advantageously between 6 and 12%.

4.  The method as claimed in any one of claims 1 to 3, **characterized in that** the drying/grinding operation is carried out at a temperature between 30°C and 90°C.

5.  The method as claimed in any one of claims 1 to 4, **characterized in that** the depolymerization of step (iii) is carried out by oxidation.

6.  The use of a carob bean gum obtained according to any one of claims 1 to 5, as thickeners, stabilizers, gelling agents (capable of forming pseudo-gels) and/or emulsifiers in the field of cosmetics, the dyeing and food industries, the oil industry and the field of cleaning products.

7.  A gelling composition comprising carob bean gum obtained according to any one of claims 1 to 5 with at least one hydrocolloid.

8.  The gelling composition as claimed in the preceding claim, **characterized in that** the hydrocolloids is chosen from xanthan gum, carragheenans, agar, Danish agar, and gellam gum.

9.  The gelling composition as claimed in either of claims 7 or 8, **characterized in that** the mass ratio of carob bean gum to the hydrocolloid(s) is between 5/95 and 95/5, advantageously between 20/80 and 80/20 and preferably between 40/60 and 60/40.

10. The gelling composition as claimed in claim 9, **characterized in that** the mass ratio of the carob bean gum to the hydrocolloid(s) is 50/50.

11. A method of gelling an aqueous gelling phase, **characterized in that** a pseudo-gel is formed by the addition of a gelling composition as claimed in any one of claims 7 to 10 to said phase, at a temperature equal to or less than 60°C, more particularly less than 60°C, advantageously between 10°C and 45°C and preferably between 15°C and 30°C, and after a sufficient rest time.

12. The method of gelling an aqueous phase as claimed in the preceding claim, **characterized in that** a pseudo-gel is formed by the addition of a gelling composition as claimed in any one of claims 7 to 10 to said phase, at a temperature varying from 20°C to 25°C.

13. A method of gelling an aqueous phase, **characterized in that** a true gel is formed by the addition of a gelling composition as claimed in any one of claims 7 to 10 to said phase, at a temperature equal to or greater than 80°C, and a sufficient rest time.

14. The use of a gelling composition as claimed in any one of claims 7 to 10, in the oil, agrochemical, food, paper and textile industries, as well as in paints and domestic or industrial cleaning agents.

15. The use of a gelling composition as claimed in claim 14 in food formulations of the following types: jellies, custards, cup custards, aspic, cold jellied poultry, bavaroises, yogurts, ice creams, sorbets, crèmes brûlées, drinks.

**Patentansprüche**

1. Verfahren zur Herstellung eines Johannisbrotgummi, umfassend die folgenden Stufen:

   i) man hydratisiert den Endosamen des Johannisbrotgummi;
   ii) man trocknet und verreibt und zerkleinert gleichzeitig den hydratisierten Endosamen;
   iii) im Anschluss an die Stufe ii) verringert man die gewichtsdurchschnittliche Molekularmasse des Johannisbrots durch dessen Depolymerisationsreaktion.

2. Verfahren gemäß Anspruch 1, **dadurch gekennzeichnet, dass** in der Stufe (i) die Hydratisierung des Endosamens des Johannisbrots eine hinreichende Zeit lang durchgeführt wird, um einen Hydratisierungsgrad zwischen 50 und 90 % zu erzielen.

3. Verfahren gemäß einem der Ansprüche 1 oder 2, **dadurch gekennzeichnet, dass** nach der Stufe ii) das Johannisbrot in Pulverform mit einem Wassergehalt vorliegt, der in vorteilhafter Weise zwischen 6 und 12 % beträgt.

4. Verfahren gemäß einem der Ansprüche 1 bis 3, **dadurch gekennzeichnet, dass** der Trocknungs/Mahlvorgang bei einer Temperatur zwischen 30 und 90°C durchgeführt wird.

5. Verfahren gemäß einem der Ansprüche 1 bis 4, **dadurch gekennzeichnet, dass** die Depolymerisationsstufe (iii) oxidativ durchgeführt wird.

6. Verwendung eines gemäß einem der Ansprüche 1 bis 5 erhaltenen Johannisbrots als Verdickungs-, Stabilisier-, Geliermittel (mit der Befähigung zur Bildung von Pseudo-Gelen) und/oder als Emulgatoren auf dem Gebiet der Kosmetik, der Nahrungsmittelfärbungsindustrien, des Erdöls und von Wartungsprodukten.

7. Gelierende Zusammensetzung, umfassend einen gemäß einem der Ansprüche 1 bis 3 erhaltenen Johannisbrotgummi zusammen mit mindestens einem Hydrokolloid.

8. Gelierende Zusammensetzung gemäß dem vorhergehenden Anspruch, **dadurch gekennzeichnet, dass** das Hydrokolloid aus Xanthan-Gummi, Carrageenanen, Agar, dänischem Agar und aus Gellan-Gummi ausgewählt ist.

9. Zusammensetzung gemäß einem der Ansprüche 7 oder 8, **dadurch gekennzeichnet, dass** das Masseverhältnis des Johannisbrotgummi zu dem oder den Hydrokolloiden zwischen 5/95 und 95/5, vorteilhaft zwischen 20/80 und 80/20 und bevorzugt zwischen 40/60 und 60/40 liegt.

10. Gelierende Zusammensetzung gemäß Anspruch 9, **dadurch gekennzeichnet, dass** das Masseverhältnis des Johannisbrotgummi zu dem oder den Hydrokolloiden 50/50 beträgt.

11. Verfahren zur Gelierung einer wässrigen Gelierphase, **dadurch gekennzeichnet, dass** man ein Pseudo-Gel durch Zugabe einer gelierenden Zusammensetzung gemäß einem der Ansprüche 7 bis 10 zur genannten Phase bei einer Temperatur gleich oder unterhalb 60°C, insbesondere unterhalb 60°C, vorteilhaft zwischen 10 und 45°C und bevorzugt zwischen 15 und 30°C nach einer hinreichenden Verweilzeit bildet.

12. Verfahren zur Gelierung einer wässrigen Phase gemäß dem vorhergehenden Anspruch, **dadurch gekennzeichnet, dass** man das Pseudo-Gel durch die Zugabe einer gelierenden Zusammensetzung gemäß einem der Ansprüche 7 bis 10 zur genannten Phase bei einer Temperatur von 20 bis 25°C bildet.

13. Verfahren zur Gelierung einer wässrigen Phase, **dadurch gekennzeichnet, dass** man ein echtes Gel durch Zugabe einer gelierenden Zusammensetzung gemäß einem der Ansprüche 7 bis 10 zur genannten Phase bei einer Temperatur gleich oder oberhalb 80°C eine hinreichende Verweilzeit lang bildet.

14. Verwendung einer gelierenden Zusammensetzung gemäß einem der Ansprüche 7 bis 10 in der Erdöl-, agrochemischen, Nahrungsmittel-, Papier- und Textil-Industrie sowie in Anstrich- und Reinigungsmitteln zum Gebrauch im Haushalt oder der Industrie.

15. Verwendung einer gelierenden Zusammensetzung gemäß Anspruch 14 in Nahrungsmittelzubereitungen von Typen von Gelees, Pudding, Karamellpudding, Aspik, Warm/Kalt-Geflügelspeisen, Cremespeisen, Joghurt, Eiscreme, Sorbets, flambierten Cremespeisen und von Getränken.

**EP 1 294 774 B1**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- Food Chemical Codex. 768